# EUROPEAN PATENT APPLICATION

(11) **EP 4 316 402 A1**
(43) Date of publication of application: **07.02.2024**
(21) Application number: 22774347.3
(22) Date of filing: 25.03.2022
(51) Int. Cl.: A61B 34/30, A61B 18/12, A61B 18/14

(54) **ELECTRIC HOOK STRUCTURE**

(30) Priority: 25.03.2021 CN 202110316576
(71) Applicant: Chengdu Borns Medical Robotics Inc., Chengdu, Sichuan 610041 (CN)
(72) Inventor: LI, Yao, Chengdu, Sichuan 610041 (CN); LI, Zhiqiang, Chengdu, Sichuan 610041 (CN)
(74) Representative: Bryn Aarflot AS
(86) International application number: PCT/CN2022/083077
(87) International publication number: WO 2022/199694

(57) **Abstract**

The invention provides an electric hook structure, wherein the electric hook head structure comprises a finger joint, a wrist joint and a forceps head base, wherein the finger joint is provided with an electric hook, one end of the wrist joint is hinged with the finger joint, the forceps head base is hinged with the other end of the wrist joint, and the finger joint and the wrist joint are in transmission connection through rope wheels. And is used for executing corresponding actions under the action of rope body tension transmission. The invention has the advantages of multiple degrees of freedom, high flexibility and high reliability.

## Description

The present invention claims priority from the Chinese application NO CN202110316576.9, the patent was submitted to the Chinese Patent Office on March 25, 2021, the patent application with the title of "an electric hook structure", Which is incorporated herein by reference in its entirety.

### Technical field

The invention relates to the technical field of robot manipulators, in particular to an electric hook structure.

### Background

As the application of robot has been widely used in various field, the operation space of robot manipulator is limited to a specific environment, so the reliability and flexibility of robot manipulator have higher requirements.

Electric hook is a energy equipment which is often used in robotic operation, and it is a form of a variety of robot manipulators. How to design a kind of electric hook structure with multi-degree of freedom, high flexibility and high reliability, and adapt it to the robot to assist operators to complete precise and complex robot operation, has become one of the main technical problems to be solved during the development process of the robots.

### Summary of the Invention

Aiming at the problems mentioned in the prior part, the invention provides an electric hook structure with multiple degrees of freedom, high flexibility and high reliability.

The present invention provides an electric hook structure. The head of that electric hook structure comprises of a finger joint, a wrist joint and a forceps head base. The electric hook is located at the finger joint, wherein the finger joint is provided with the electric hook, one end of the wrist joint is hinged with the finger joint, the forceps head base is hinged with the other end of the wrist joint, and the finger joint and the wrist joint are in transmission connection through rope wheels, it is used for executing corresponding actions under the action of rope body tension transmission.

In one embodiment, the plane of motion of the finger joint and the plane of motion of the wrist joint are perpendicular to each other.

The embodiment is beneficial that the electric hook can move in multiple degrees of freedom.

In one embodiment, the finger joint comprises a forceps head, a first guide wheel, a finger joint rotating shaft and a first twist rope, wherein the first guide wheel is integrally arranged on the forceps head, the finger joint rotating shaft penetrates through the first guide wheel and is fixed on the wrist joint, and the first guide wheel can rotate relative to the finger joint rotating shaft, the first twist rope rounds the first guide wheel and is in transmission connection with the rope wheel of the first guide wheel. And that first twist rope is used for drive the first guide wheel to rotate so as to realize the deflection of the electric hook, wherein one end of the electric hook is inserted in the forceps head and fixedly connected with the forceps head.

The above embodiment has the following benefits: through the transmission connection between the first twist rope and the rope wheel of the first guide wheel, under the action of the rope body tension, the first twist rope drives the first guide wheel to rotate to realize the deflection of the electric hook.

In one embodiment, the wrist joint comprises a wrist joint body, a second guide wheel, a wrist joint rotating shaft and a second twist rope. One end of the wrist joint body is connected with the finger joint rotating shaft, the second guide wheel is integrally arranged at the other end of the wrist joint body, and the wrist joint rotating shaft penetrates through the second guide wheel and is fixed on the forceps head base. The second guide wheel and the wrist joint rotating shaft can rotate relatively, a second twist rope rounds the second guide wheel and is in transmission connection with the rope wheel of the second guide wheel; That second twist rope is used for drive the second guide wheel to rotate to realize the rotation of the wrist joint.

The above embodiment has the beneficial effect that the second twist rope is in transmission connection with the rope wheel of the second guide wheel, and the second twist rope drives the second guide wheel to rotate under the action of the rope body tension to realize the rotation of the wrist joint.

In one embodiment, the electric hook structure comprises at least two groups of pulley blocks, which are respectively arranged on the wrist joint and/or the forceps head base, and the two ends of the first twist rope are respectively wound around the corresponding pulley blocks for reversing and tensioning the first twist rope.

The beneficial effect of the above embodiment is that the pulley block is used for reversing and tensioning the first twist rope.

In one embodiment, the electric hook structure comprises a lead, one end of which is inserted in the forceps head and is electrically connected with the electric hook.

The beneficial effect of the above embodiment is that the electric hook is connected with the power supply system through a wire to provide energy for the operation of the electric hook.

In one embodiment, the wires are spirally arranged inside the forceps head.

The above embodiment has the advantage of preventing the wire from being broken by external force.

In one embodiment, the wrist joint rotating shaft is provided with a guide wheel for reversing the first twist rope and/or the wire.

The beneficial effect of the above embodiment is that the guide wheel is used for reversing the first twist rope and/or the wire.

In one embodiment, the first twist rope and the second twist rope are both ropes having the characteristics of tensile resistance, impact resistance, wear resistance, flexibility, etc. Steel wire ropes are preferred in the present invention.

The above embodiment has the beneficial effects that the steel wire rope can transmit long-distance load, the bearing safety factor is large, and the use is safe and reliable.

Compared with the prior art, the present invention has the following advantages:
(1) Multiple degrees of freedom, high flexibility, and high reliability.
(2) Simple structure and convenient operation.
(3) The rope pulley transmission connection mode is adopted, and the transmission is stable without noise, vibration and impact.

The features described above may be combined in any suitable manner or replaced by equivalent features so long as the objectives of the present invention are achieved.

### Brief Description of the Drawings

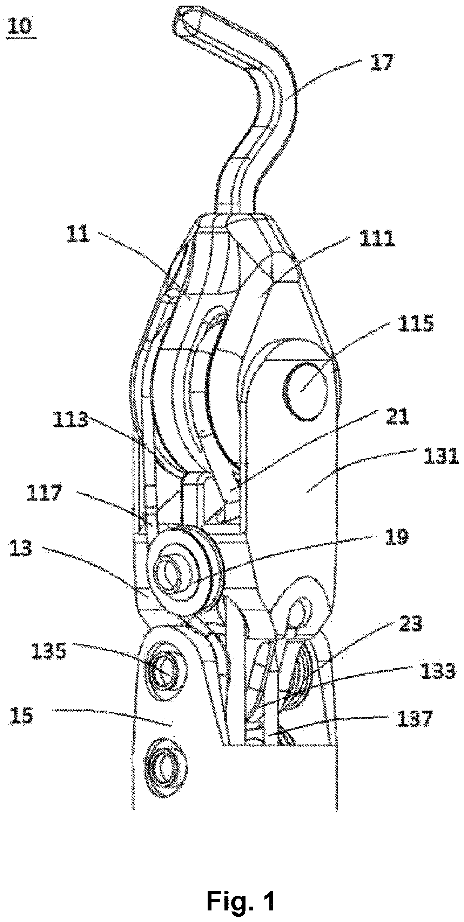

Hereinafter, the present invention will be described in more detail on the basis of examples and with reference to the accompanying drawings. Among
Fig. 1 show a first isometric view of that head structure of an electrical hook;

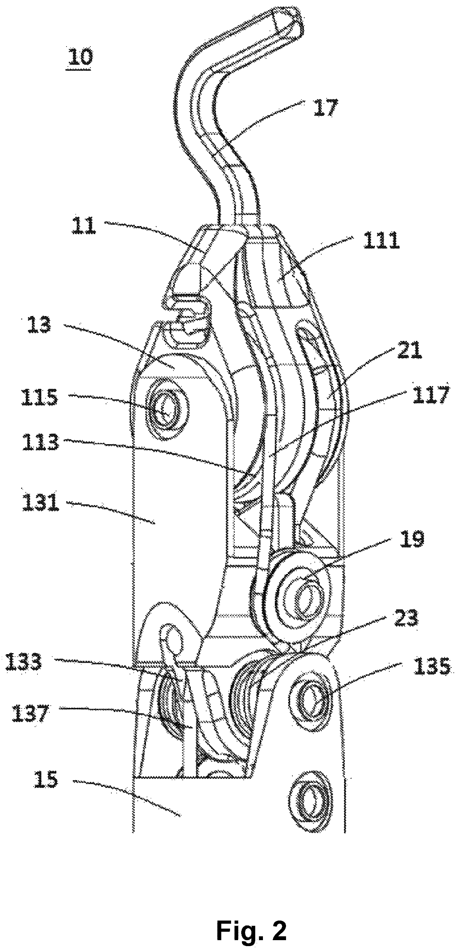

Fig. 2 show a second isometric view of that head structure of an electrical hook;

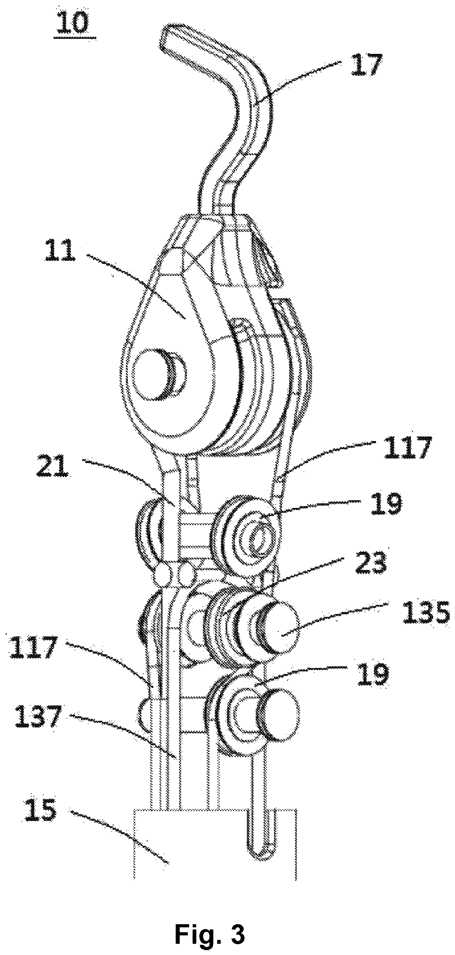

Fig. 3 shows the first part of the head structure of an electrical hook;

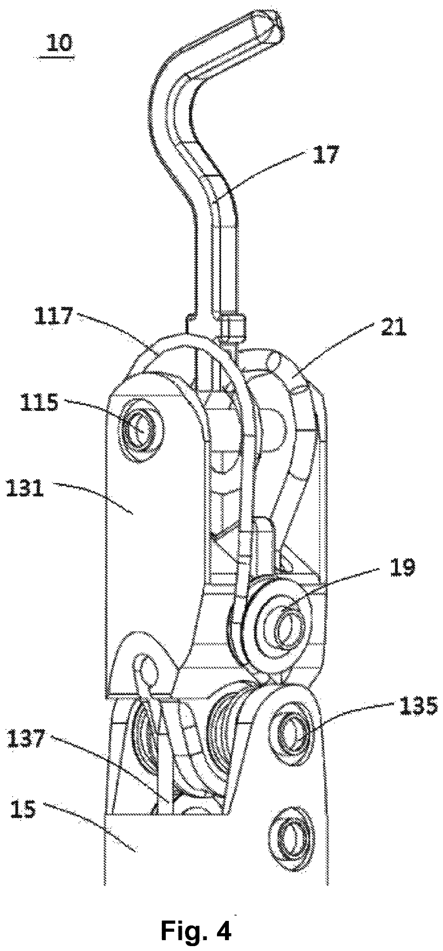

Figure 4 shows the second part of the head structure of an electrical hook;

In the drawings, like parts are given like reference numerals. The drawings are not to scale.

10- Head structure of an electrical hook; 11- Finger joint; 111- Forceps head; 113-First guide wheel; 115- Finger joint rotating shaft; 117- First twist rope; 13- Wrist joint; 131-Wrist joint body; 133- Second guide wheel; 135- Wrist joint rotating shaft; 137- Second twist rope; 15- Forceps head base; 17- Electric hook; 19- Pulley block; 21- guide wire; 23-guide wheel.

### Detailed Description of the Invention

In order that the present invention may be more fully understood, the present invention will be described more comprehensively with reference to the relevant drawings. Preferred embodiments of the present invention are illustrated in the accompanying drawings. This invention may, however, be embodied in many different forms and is not limited to the embodiments described herein.

An head structure of an electrical hook 10 comprises a finger joint 11, a wrist joint 13 and a forceps head base 15, wherein an electric hook 17 is arranged on the finger joint 11, one end of the wrist joint 13 is hinged with the finger joint 11, the forceps head base 15 is hinged with the other end of the wrist joint 13, and both the finger joint 11 and the wrist joints 13 are in transmission connection by a rope pulley. The movement plane of the finger joint 11 is perpendicular to the movement plane of the wrist joint 13.

Specifically, in this embodiment, the electric hook 17 is a hook-shaped structure with a certain curvature, and can complete electrocoagulation cutting and hemostasis in the electrified state.

The finger joint 11 comprises a forceps head 111, a first guide wheel 113, a finger joint rotating shaft 115 and a first twist rope 117. The first guide wheel 113 is integrally arranged on the forceps head 111, and the finger joint rotating shaft 115 penetrates through the first guide wheel 113 and is fixed on the wrist joint 13. The first guide wheel 113 and the finger joint rotating shaft 115 can rotate relative to each other, the first twist rope 117 rounds the first guide wheel 113 and is in transmission connection with the rope wheel of the first guide wheel 113, the first twist rope 117 is used for driving the first guide wheel 113 to rotate so as to realize the deflection of the electric hook 17, one end of the electric hook 17 is inserted in the forceps head 111, And is fixedly connected with that forceps head 111.

Specifically, in this embodiment, the forceps head 111 is formed by combining a first forceps head and a second forceps head. The first forceps head and the second forceps head are connected together by ultrasonic welding. The end of the electric hook 17 connected with the forceps head 111 is provided with a stop block, the end of the electric hook 17 provided with the stop block is inserted into the forceps head 111, and the first forceps head and the second forceps head are combined to wrap the end provided with the stop block, so as to realize the fixed connection between the electric hook 17 and the forceps head 111.

The first guide wheel 113 is integrally arranged on the first forceps head, the finger joint rotating shaft 115 penetrates through the first guide wheel 113 and is fixed on the wrist joint 13, the first guide wheel 113 and the finger joint rotating shaft 115 can rotate relatively, one point of the first twist rope 117 is fixed on the first forceps head, the first twist rope 117 passes around the first guide wheel 113 and is in transmission connection with the rope wheel of the first guide wheel 113, so as to drive the first guide wheel 113 to rotate, thereby realizing the deflection of the electric hook 17.

The two ends of the first twist rope 117 are respectively wound on the same drive shaft of the first drive mechanism clockwise and counterclockwise or on the drive shaft that moves synchronously, and the drive shaft of the first drive mechanism can pull the first twist rope 117 to move during rotation. The first twist rope 117 drives the first forceps head (i.e., the forceps head 111) to rotate around the finger joint rotating shaft 115 during the movement, thereby realizing the deflection of the electric hook 17.

In one embodiment, finger joint rotating shaft 115 is rotationally coupled to wrist joint 13.

In one embodiment, the end of the electric hook 17 provided with the stop block is further provided with a connecting ring, and the connecting ring is sleeved on the finger joint rotating shaft 115 to prevent the electric hook 17 from falling off.

The wrist joint 13 comprises a wrist joint body 131, a second guide wheel 133, a wrist joint rotating shaft 135 and a second twist rope 137. One end of the wrist joint body 131 is connected with the finger joint rotating shaft 115, and the second guide wheel 133 is integrally arranged at the other end of the wrist joint body 131. A wrist joint rotating shaft 135 passe through that second guide wheel 133 and is fixed on the forceps head base 15, the second guide wheel 133 and the wrist joint rotating shaft 135 can rotate relatively, a second twist rope 137 rounds the second guide wheel 133 and is in transmission connection with the rope wheel of the second guide wheel 133, and the second twist rope 137 is used for driving the second guide wheel 133 to rotate, To effect rotation of the wrist joint 13.

Specifically, in this embodiment, one end of the wrist joint body 131 is fixedly connected or rotatably connected with the finger joint rotating shaft 115, the second guide wheel 133 is integrally arranged on the other end of the wrist joint body 131, and the wrist joint rotating shaft 135 passes through the second guide wheel 133 and is fixed on the forceps head base 15. The second guide wheel 133 and the wrist joint rotating shaft 135 can rotate relative to each other, and the second twist rope 137 passes around the second guide wheel 133 and is in transmission connection with the rope wheel of the second guide wheel 133, so as to drive the second guide wheel 133 to rotate, thereby realizing the rotation of the wrist joint 13.

The two ends of the second twist rope 137 are respectively wound on the same drive shaft of the second drive mechanism clockwise and counterclockwise or on the drive shaft that moves synchronously, the drive shaft of the second drive mechanism can pull the second twist rope 137 to move in the process of rotation, and the second twist rope 137 can drive the wrist joint body 131 to rotate around the wrist joint rotating shaft 135 in the process of movement. Thereby effecting rotation of the wrist joint 13.

In one embodiment, the wrist joint rotating shaft 135 is rotationally connected to the forceps head base 15.

Wherein, the head structure of an electrical hook 10 further comprises at least two groups of pulley blocks 19, the at least two groups of pulley blocks 19 are respectively arranged on the wrist joint 13 and/or the forceps head base 15, both ends of the first twist rope 117 are respectively wound around the corresponding pulley block 19 for reversing and tensioning the first twist rope 117, The reversing and tensioning of the first twist rope 117 can prevent the first twist rope from loosening during movement and can follow a predetermined path.

Specifically, in this embodiment, the head structure of an electrical hook 10 further includes two groups of pulley blocks 19, the two groups of pulley blocks 19 are respectively located on both sides of the wrist joint 13. Each group of pulley block 19 includes two fixed pulleys, one of which is fixed on the wrist joint 13, and the other fixed pulley is fixed on the forceps head base 15. One end of the first twist rope 117 is wound around the two fixed pulleys to realize the direction change and tension of the first twist rope 117, and the other end of the first stranded rope 117 is the same as that described above and is not described in detail. The two ends of the first twist rope 117 after reversing are respectively located on the two sides of the wrist joint rotating shaft 135, so as to ensure that the first twist rope 117 will not pull the wrist joint 13 to be dead and cause it unable to move.

In one of the embodiments, the two sets of pulley blocks 19 are respectively disposed on two opposite sides of the wrist joint 13 or the forceps head base 15, and the two ends of the first twist rope 117 are respectively wound around a corresponding one of the pulley blocks 19 for reversing and tensioning the first twist rope 117.

The head structure of an electrical hook 10 further includes a guide wire 21. One end of the guide wire 21 is inserted into the forceps head 111 and is electrically connected to the electric hook 17.

Specifically, in this embodiment, one end of the guide wire 21 is inserted into the forceps head 111 and electrically connected to the electric hook 17, the guide wire 21 is arranged in a spiral manner inside the forceps head 111, and the first forceps head and the second forceps head are spliced to wrap the end of the electric hook 17 provided with the stop block and the guide wire 21 inserted into the forceps head 111.

In one embodiment, the second forceps head is provided with a guide groove, and the guide wire 21 extend from the second forceps head and are arranged along the guide groove.

The wrist joint rotating shaft 135 is provided with a guide wheel 23 for reversing the first twist rope 117 and/or the guide wire 21.

Specifically, in this embodiment, the two ends of the wrist joint rotating shaft 135 are respectively rotatably provided with a guide wheel 23, the two guide wheels 23 are respectively located between the two fixed pulleys of each pulley block 19, and the first twist rope 117 and/or the guide wire 21 are wound around the guide wheel 23 for direction change of the first twist rope 117 and/or the guide wire 21.

In this embodiment, both the first twist rope 117 and the second twist rope 137 are made of steel wire ropes, and the steel wire ropes can transmit long-distance loads, having a large bearing safety factor, and are safe and reliable to use.

In the description of the present invention, it should be understood that the orientations or positional relationships indicated by the terms "upper," lower, "bottom," top, "front," rear, "inner, outer, left, right, and the like are based on the orientations or positional relationships shown in the drawings only for convenience of description of the present invention and simplification of description, It is not intended to indicate or imply that the devices or elements referred to must have a particular orientation, be constructed or operate in a particular orientation, and therefore, should not be construed as limiting the invention.

Although the present invention has been described herein with reference to particular embodiments, it is to be understood that these embodiments are merely illustrative of the principles and applications of the present invention. It is therefore to be understood that many modifications may be made to the exemplary embodiments and that other arrangements may be devised without departing from the spirit and scope of the invention as defined by the appended claims. It will be appreciated that different dependent claims and features described herein may be combined in ways other than those described in the original claims. It will also be appreciated that features described in connection with a single embodiment may be used in other described embodiments.

## Claims

1. A electric hook structure, comprises of a finger joint, a wrist joint and a forceps head base, the finger joint is provided with the electric hook, one end of the wrist joint is hinged with the finger joint, the forceps head base is hinged with the other end of the wrist joint, and the finger joint and the wrist joint are in transmission connection through rope wheels, it is used for executing corresponding actions under the action of rope body tension transmission.

2. The electric hook structure according to claim 1, wherein the plane of motion of the finger joint and the plane of motion of the wrist joint are perpendicular to each othe.

3. The electric hook structure according to claim 2, wherein the finger joint comprises a forceps head, a first guide wheel, a finger joint rotating shaft and a first twist rope, wherein the first guide wheel is arranged on the forceps head, the finger joint rotating shaft penetrates through the first guide wheel and is fixed on the wrist joint, and the first guide wheel can rotate relative to the finger joint rotating shaft, the first twist rope rounds the first guide wheel and is in transmission connection with the rope wheel of the first guide wheel; and that first twist rope is used for drive the first guide wheel to rotate so as to realize the deflection of the electric hook, wherein one end of the electric hook is inserted in the forceps head and fixedly connected with the forceps head.

4. The electric hook structure according to claim 3, wherein the wrist joint comprises a wrist joint body, a second guide wheel, a wrist joint rotating shaft and a second twist rope; one end of the wrist joint body is connected with the finger joint rotating shaft, the second guide wheel is integrally arranged at the other end of the wrist joint body, and the wrist joint rotating shaft penetrates through the second guide wheel and is fixed on the forceps head base; the second guide wheel and the wrist joint rotating shaft can rotate relatively, a second twist rope rounds the second guide wheel and is in transmission connection with the rope wheel of the second guide wheel; That second twist rope is used for drive the second guide wheel to rotate to realize the rotation of the wrist joint.

5. The electric hook structure according to claim 4, wherein the electric hook structure comprises at least two groups of pulley blocks, which are respectively arranged on the wrist joint and/or the forceps head base, and the two ends of the first twist rope are respectively wound around the corresponding pulley blocks for reversing and tensioning the first twist rope.

6. The electric hook structure according to claim 5, wherein the electric hook structure comprises a lead, one end of which is inserted in the forceps head and is electrically connected with the electric hook.

7. The electric hook structure according to claim 6, wherein the wires are spirally arranged inside the forceps head.

8. The electric hook structure according to claim 6, wherein the wrist joint rotating shaft is provided with a guide wheel for reversing the first twist rope and/or the wire.

9. The electric hook structure according to claim 4, wherein the first twist rope and the second twist rope both use steel wire ropes.

10. The electric hook structure according to claim 7, wherein the electric hook is an arc-shaped structure.
